# EUROPEAN PATENT SPECIFICATION

(11) **EP 0 392 326 B1**
(45) Date of publication and mention of the grant of the patent: **20.07.1994**
(21) Application number: 90106404.8
(22) Date of filing: 04.04.1990
(51) Int. Cl.: C07D 233/56, A61K 31/415

(54) **Dichloro-substituted imidazole derivatives as antifungal agents**
Dichloro-substituierte Imidazol-Derivate als antifungizide Mittel
Dérivés d'imidazolo dichloro substitués comme agents fongicides

(30) Priority: 10.04.1989 ES 8901237
(43) Date of publication of application: 17.10.1990
(73) Proprietor: SOCIEDAD ESPANOLA DE ESPECIALIDADES FARMACO-TERAPEUTICAS, S.A., E-08026 Barcelona (ES)
(72) Inventor: Andreoli Rovati, Romeo, E-08023 Barcelona (ES); Cepero Mestres, Ricardo, E-08004 Barcelona (ES)
(74) Representative: Rambelli, Paolo

(56) References cited:
- WO-A-88/03138
- GB-A- 1 593 417

## Description

This invention refers to the two new dichloro-substituted 1-(5H-dibenzo[a,d]cyclohepten-5-yl)-1H-imidazole compounds of formula (I), in which -X- is either -CH₂-CH₂- or -CH=CH-, and their pharmaceutically acceptable addition salts, a process for their production, and their use in the preparation of antifungal pharmaceutical and veterinary compositions.

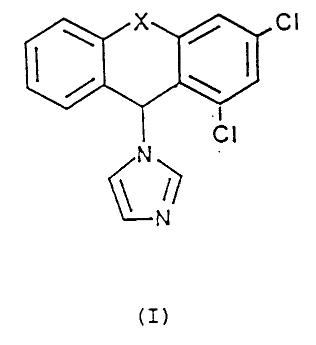

### BACKGROUND ART

It is known that N-tritylimidazole derivatives show fungicide activities. For instance, US 3.660.577 discloses antimycotics of the general formula (II), wherein R, R₁ and R₂ denote hydrogen, lower alkyl radicals and phenyl; X stands for alkyl groups or for electronegative substituents; and n denotes an integer from 0 to 2, and n may have different meanings in the individual benzene rings. 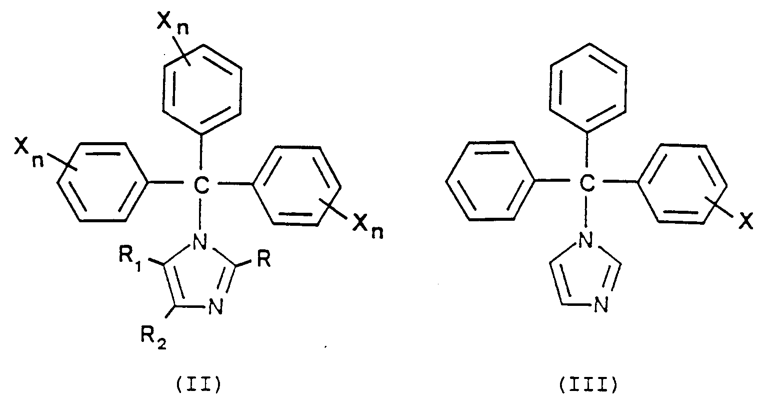
It known that, in general, among the very many products embraced by general formula (II), mono-substituted ones are more active as antifungal agents than poly-substituted ones. Thus, for instance, FR 1.600.990 discloses antimycotics of general formula (III), where X stands for several substituents, halogens included. This document describes comparative tests of mono-substituted halogen derivatives versus analogous di-and tri-substituted ones; from the results it is clear that mono-substituted derivatives have higher antifungal activities. In fact, the most successful commercial product included in general formula (III) is the mono-substituted chloro derivative named Clotrimazole. 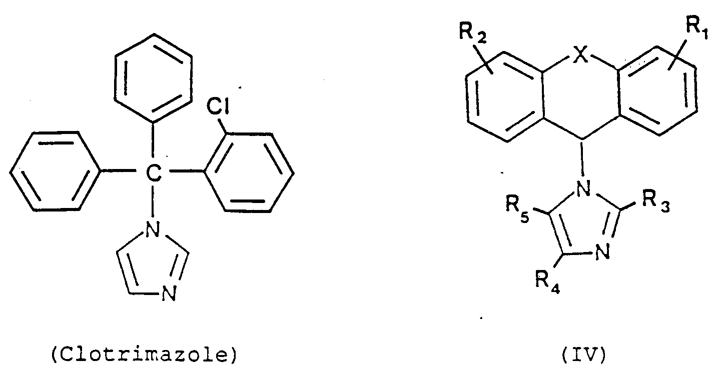
US 3.764.609 disclosed 1-(5H-dibenzo[a,d]cyclohepten-5-yl)-1H-imidazole derivatives of general formula (IV), wherein R₁ and R ₂ are the same or different and each represents a hydrogen or halogen atom or a lower alkyl group, R₃, R₄ and R₅ represent a hydrogen atom or an alkyl group, X represents a single bond, a -CH₂-CH₂-, -CH₂-CH₂-CH₂- or -CH=CH- group or a group -CH₂-CHHal- wherein Hal represents a chlorine or bromine atom. It is said that products of general formula (IV) possess fungicidal activity, although no data is given in this document. It is noteworthy that general formula (IV) does not embrace any product di-substituted in the same phenyl ring. Actually no di-substituted product is described in this document, even with substitution in different rings. Among the described products in this patent, the one which is structurally closest to those of the present invention is the product of formula (V), which has a bromine atom in position 3. 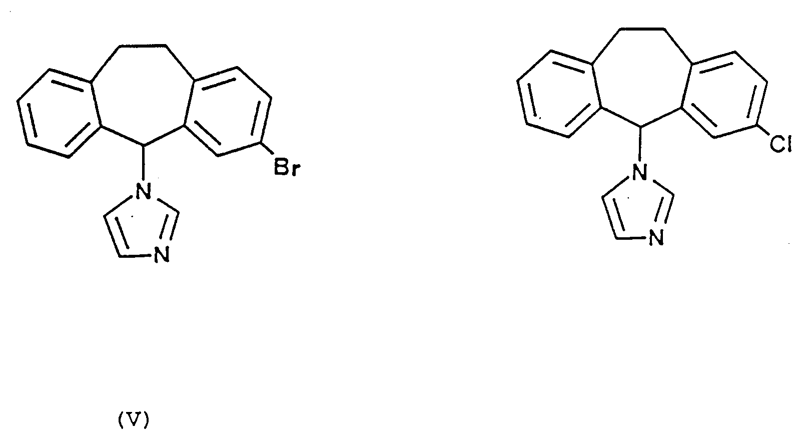
Squibb patent US 4.420.474 discloses synergistic antifungal compositions of antimycotics and Azalomycin F. In its Table I 1-(3-chloro-10,11-dihydro-5H-dibenzo[a,d]dicyclohepten-5-yl)-1H-imidazole is mentioned with the company name SQ 80896, but without giving any indication for its preparation or use. To the best of our knowledge, SQ 80896 is structurally the closest product to the products of the present invention, ever described. In any case, the pharmacological data in Table I of patent US 4.420.474 clearly show that SQ 80896 is considerably less active than Clotrimazole (according to MIC parameters against Candida albicans).

Despite the usefulness of the already known commercial antifungal agents, research in this field is very intensive because some fungi species have become resistant to old compounds (particularly to the widespread Clotrimazole). This represents a serious problem, specially in hospitals and big towns. It is very important to search for new antifungals with powerful action and wide spectrum; but it is also important to provide new antifungals active against a limited number of fungal infections, some of which could be resistant to old products. Thus, new compounds (Econazole, Miconazole,.. and notably Bifonazole) are being introduced in the market as soon as they show any antifungal activity against some species similar to, or higher than, the activity of old products, notably of Clotrimazole which is still the most active commercial product against some species.

### DISCLOSURE OF THE INVENTION

The above-mentioned state of the art clearly shows that there was a general trend pointing away from the screening of poly-halogen substituted compounds, in the search for new imidazole derivatives with antifungal activity. This tendency was very clear for N-tritylimidazole type of general formula (II), as illustrated by the teaching of the above-mentioned patent FR 1.600.990.

We have observed a similar trend in the course of our own research. Thus, for instance, we have prepared product WAS 2162 (as a nitric acid salt) and we have found that it is considerably less active than Clotrimazole, as illustrated by the results of the Table of Example 3. 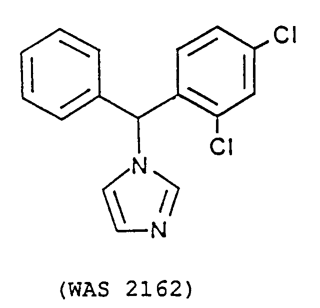
Substitution of imidazole derivatives of the(dibenzo[a,d]cyclohepten-5-yl)-1H-imidazole type (general formula IV) for the classical N-tritylimidazole derivatives (general formula III) did not appear a promising trend in the field of antimycotics. Actually, to our knowledge, no derivative of those included in general formula (IV) has ever been marketed. As mentioned above, US 4.420.474 patent mentions that SQ 80896 is considerably less active than Clotrimazole. We have also prepared and tested SQ 80896 and, according to our experience (cfr. e.g. the table of Example 3), this product is considerably less active than Clotrimazole.

Despite the two trends mentioned above, we have unexpectedly found that dichloro-substituted 1-(5H-dibenzo[a,d]cyclohepten-5-yl)-1H-imidazole compounds of formula (I), where -X- is either a -CH₂-CH₂- or a -CH=CH- group, and their pharmaceutically acceptable salts, surprisingly have a antifungal activity comparable -or superior- to that of some antifungals on the market, in particular Clotrimazole and Bifonazole. This is clearly illustrated in the pharmacological comparative test of Examples 3-7. Thus, according to one main aspect of the invention, new products of formula (I) are provided to fight against fungal infection. As products (I) belong to a chemical class never marketed before, it is almost sure that many fungi species have not developed resistance to them, what constitutes an advantage over other already-used products.

The number and position of chloro substitution, together with the lack of mobility provided to the molecule by the -CH₂-CH₂- or the -CH=CH- bridges, are two simultaneous structural features which are essential for the antifungal activity. Thus, if the chloro substitution is different (as is product SQ 80896), or if the bridge disappears (as in product WAS 2162), the antifungal activity dramatically diminishes.

General formula (I) embraces two main embodiments of the invention, namely, compound 1-(2,4-dichloro-10,11-dihydro-5H-dibenzo[a,d]cyclohepten-5-yl)-1H- imidazole and its pharmaceutical acceptable salts, and compound 1-(2,4-dichloro-5H-dibenzo[a,d]cyclohepten-5-yl)-1H-imidazole and its pharmaceutical acceptable salts. Their nitric acid salts, prepared and described here for the first time, are respectively denoted WAS 2160 and WAS 2169 in the following. The names and formulas used here represent racemic mixtures. Although it is expected than one enantiomer will have more activity than its mirror-image one, all stereoisomers and their mixtures are to be considered subject-matter of the present invention. 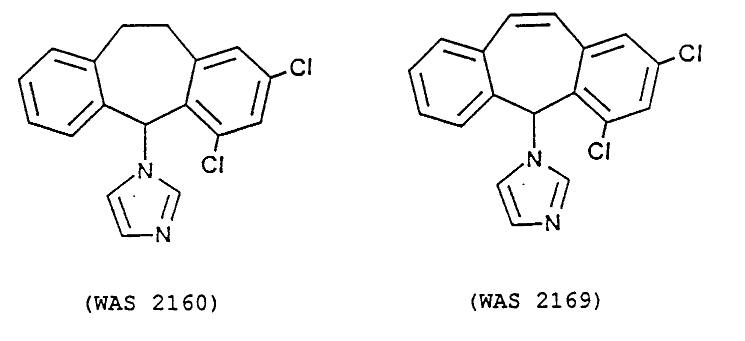
Another main aspect of the invention are pharmaceutical compositions for treating fungal infections in humans and animals which comprises an antifungal effective amount of a compound of formula (I) or a pharmaceutically acceptable salt thereof, in combination with a pharmaceutically acceptable excipient or carrier. A preliminary test in rabbits (cfr. Example 8) clearly shows that products of the invention are innocuous and totally tolerated by the eye, the skin and the vagina.

The invention also provides the use of a compound of formula (I) or a pharmaceutically acceptable salt thereof in the preparation of a medicament for treating fungal infections in humans or animals.

Pharmaceutically acceptable salts of the products of the present invention are those obtained from physiologically tolerated inorganic acids, such as nitric, phosphoric, sulphuric or hydrochloric, sulphonic acids, and carboxylic or hidroxycarboxylic acids, such as acetic, tartaric, salicylic, citric, ascorbic, etc. In a preferred embodiment, the salt is the one obtained from nitric acid.

By pharmaceutically acceptable excipients or carriers there are to be understood solid, semi-solid or liquid diluents, fillers and formulation auxiliaries of all kinds. Tablet, pills, capsules, granules, solutions, suspensions, emulsions, suppositories, pastes, ointments, gels, creams, lotions, powders and sprays containing products of formula (I) may be mentioned as possible pharmaceutical compositions.

Another main aspect of the invention is a process for preparing a compound of formula (I) or a pharmaceutically acceptable salt thereof, which comprises reacting a compound of formula (VI), where Z is a halogen group or a sulphonate group (e.g. methanesulphonate, benzenesulphonate or tosylate), with imidazole or an equivalent nucleophilic reactant (e.g. an imidazole salt or trimethylsilylimidazole), in a polar inert solvent (e.g. dimethylformamide), in the presence of a base (e.g. an excess of imidazole, triethylamine, potassium carbonate or equivalent) to neutralise the acid formed; and, if a salt is desired, the subsequent addition of the corresponding acid. 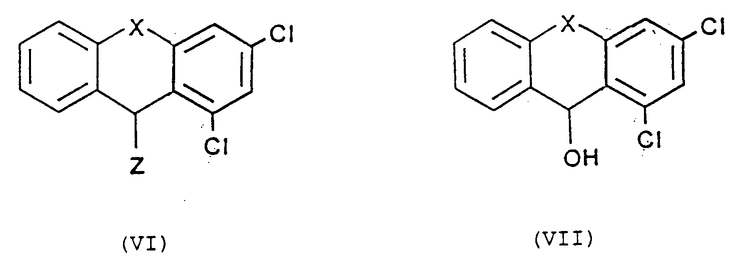
Preferred embodiments of the invention are the following: Z is Cl in intermediate (VI), this being obtained from (VII) with thionyl chloride; the reaction is carried out in excess of imidazole, without addition of another base, and in dimethylformamide as solvent; alcohols (VII) are obtained from ketones (VIII) by standard reduction methods such a catalytic hydrogenation or treatment with hydrides, preferably with sodium borohydride. 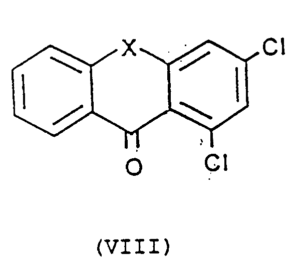
The nucleophilic substitution reactions of the process of the present invention have the particular difficulty of the presence of an steric hindrance due to the great proximity of the chloro substituent nearest to the reaction center. However, under the preferred conditions of this invention (excess of imidazole, in dimethylformamide under reflux, for 6 h, with subsequent addition of nitric acid), yields are high.

Products of formula (VI) can be prepared by treatment of alcohols of formula (VII) with standard reagents of Organic Chemistry. Thus, e.g., when Z is Cl, thionyl chloride can be used; when Z is Br, phosphorous tribromide in pyridine can be used; when Z is tosylate, tosyl chloride can be used; etc.

Alcohols of formula (VII) can be prepared by reduction of the corresponding ketones by standard organic synthetic methods, such as treatment with sodium borohydride or by catalytic hydrogenation. The two ketones (VIII) required for the preparation of the products of the present invention have been described in the literature (cfr. X. Cirera, PhD Dissertation, Instituto Químico de Sarriá, Barcelona, 1983). Final products (I) and intermediates (VII, and VI with Z=Cl) have not been described before.

The present invention is illustrated by the following examples.

### EXAMPLE 1

### 1-(2,4-Dichloro-10,11-dihydro-5H-dibenzo[a,d]cyclohepten-5-yl)-1H-imidazole nitrate (WAS 2160)

a) A solution of 50 mmol of 2,4-dichloro-10,11-dihydro-5H-dibenzo[a,d]cyclohepten-5-one in 100 mL of methanol was placed in a 250-mL flask provided with magnetic stirring, reflux condenser and addition funnel. Then, a solution of 50 mmol of sodium borohydride in 5 mL of water and 15 mL of methanol was added during 15 min. The mixture was shaken at room temperature for three hours and it was brought to pH=1 with concentrated hydrochloric acid. The solvent was removed under vacuum. The residue was dissolved in 75 mL of chloroform and washed with 2 x 50 mL of water. The organic phase was dried with anhydrous magnesium sulphate and filtered. The solvent was removed under vacuum. The residue was used without more purification in the next step. I.R. (KBr): 3500-3200, 3020, 2940, 1590, 990 cm⁻¹. The disappearance of the carbonyl band was observed.
b) The crude material from the previous step was dissolved in 100 mL of methylene chloride. To this solution 75 mmol of thionyl chloride were added at room temperature for five minutes. The solvent was removed under vacuum and the residue was dissolved in 50 mL of thionyl chloride and heated under reflux for an hour. The excess thionyl chloride was distilled under vacuum; 50 mL of toluene were added; the solvent was removed under vacuum and the residue was used without more purification in the next step. I.R. (KBr): 3030-3010, 2940, 1585 cm⁻¹. The disappearance of the bands corresponding to hydroxyl stretching was observed.
c) The crude material from the previous step was dissolved in 100 mL of dimethylformamide. Then 200 mmol of imidazole were added and the mixture was heated under reflux for six hours. The solvent was removed under vacuum; the residue was dissolved in 75 mL of chloroform and washed with 3 x 50 mL of water. The organic phase was dried with anhydrous magnesium sulphate and filtered. The solvent was removed under vacuum. The residue was dissolved in 50 mL of isopropyl ether and 25 mL of isopropyl alcohol. Then 60% (density=1.38) nitric acid was added until total precipitation of the title compound was reached. After filtration, the solid was recrystallised from isopropyl ether/isopropyl alcohol. Overall yield: 56%. M.p. 173-174°C. I.R. (KBr) : 3160, 3100, 3000-2400 (N-H), 1590, 1400, 1320, 1290, 895, 860 cm⁻¹; ¹H-RMN (DCCl₃/CD₃OD): δ=8.2/s(1H), 7.5-6.7/sc(9H), 2.9-2.6/sc(4H).

### EXAMPLE 2

### 1-(2,4-dichloro-5H-dibenzo[a,d] cyclohepten-5-yl)-1H-imidazole nitrate (WAS 2169)

a) A solution of 50 mmol of 2,4-dichloro-5H-dibenzo[a,d]cyclohepten-5-one in 100 mL of methanol was placed in a 250-mL flask provided with magnetic stirring, reflux condenser and addition funnel. A solution of 50 mmol of sodium borohydride in 5 mL of water and 15 mL of methanol was added for 15 min. The mixture was shaken at room temperature for three hours and brought to pH=1 with concentrated hydrochloric acid. The solvent was removed under vacuum. The residue was dissolved in 75 mL of chloroform and washed with 2 x 50 mL of water. The organic phase was dried with anhydrous magnesium sulphate and filtered. The solvent was removed under vacuum, and the residue was used without more purification in the next step. I.R. (KBr): 3500-3200, 3020, 1595, 1000 cm-1. The disappearance of the carbonyl band was observed.
b) The crude material from the previous step was dissolved in 100 mL of methylene chloride. Then 75 mmol of thionyl chloride were added at room temperature for five minutes. The solvent was removed under vacuum and the residue was dissolved in 50 mL of thionyl chloride. The mixture was heated under reflux for an hour. The excess of thionyl chloride was distilled under vacuum; 50 mL of toluene were added; the solvent was removed under vacuum and the residue was used without more purification in the next step. I.R. (KBr): 3030-3000, 2940, 1580 cm⁻¹. The disappearance of the bands corresponding to the stretching of the hydroxyl group was observed.
c) The crude material from the previous step was dissolved in 100 mL of dimethylformamide. Then 200 mmol of imidazole were added and the mixture was heated under reflux for six hours. The solvent was removed under vacuum. The residue was dissolved in 75 mL of chloroform and washed with 3 x 50 mL of water. The organic phase was dried with anhydrous magnesium sulphate and filtered. The solvent was removed under vacuum. The residue was collected in 50 mL of isopropyl ether and 25 mL of isopropyl alcohol. Then 60% (density=1.38) nitric acid was added until total precipitation of the title compound was reached. After filtration, the solid was recrystallised from isopropyl ether/isopropyl alcohol. Overall yield: 48%. M.p.: 165-167ºC, I.R. (KBr): 3160, 3000-2300 (N-H), 1580, 1500, 1320, 1295, 890, 850, 750 cm⁻¹. ¹H-RMN (DCCl₃/CD₃OD): δ=7.75/s(1H), 7.6-7.1/sc(9H), 6.8/d(1H), 6.5/m(1H).

### EXAMPLE 3

### In vitro activity against Candida tropicalis CECT 1440.

This pharmacological test allows a comparison of the activities of the two products of the present invention (WAS 2160 and WAS 2169) with the activities of the structurally closest described product (SQ 80896), the structurally closest commercial product (Clotrimazole) and the analogue product without the seven-membered ring (WAS 2162). MIC (Minimum Inhibitory Concentration, in fg/mL) and RIF (Relative Inhibition Factor) parameters were determined in a standard way, obtaining the results of the table (WAS 2162 is the nitric acid salt of 1-[(2,4-dichlorophenyl)phenylmethyl]-1H-imidazole, prepared at our laboratory).

| Candida tropicalis | | |
|---|---|---|
| Product | MIC | RIF |
| WAS 2160 | 7.79 | 39.31 |
| WAS 2169 | 16.13 | 54.94 |
| SQ 80896 | >100 | 83.96 |
| WAS 2162 | >100 | 94.47 |
| Clotrimazole | 11.81 | 43.05 |

### EXAMPLE 4

### In vitro ativity against 116 strains of yeasts and 45 strains of dermatophytes

MIC-s were determined by the method of progressive double dilutions in a liquid medium, using sterile microplates. The culture medium used was the Sabouraud broth with gentamycin (100 mg/1000 mL). The results obtained in comparative tests with WAS 2169, WAS 2160 and Clotrimazole (Clot.) are shown in the following table.

| Yeasts and Dermatophytes | | | | |
|---|---|---|---|---|
| | WAS 2160 | WAS 2169 | CLOT. | BIF. |
| C. albicans | 2,25 | 1,98 | 2,43 | 12,10 |
| C. tropicalis | 3,44 | 7,58 | 3,51 | 72,67 |
| C. parapsilosis | 2,06 | 1,08 | 2,36 | 58,97 |
| C. Krusei | 3,17 | 5,61 | 6,18 | 50,50 |
| Torulopsis glabrata | 1,25 | 6,33 | 6,29 | 14,88 |
| Microsporum canis | 0,39 | 0,06 | 2,69 | 3,12 |
| Trichoph. mentagrophytes | 0,17 | 0,17 | 0,23 | 1,48 |
| Trich. rubrum | 0,54 | 0,30 | 0,31 | 1,19 |
| M. gypseum | 1,05 | 0,08 | 0,97 | 6,25 |
| Epidermophyton floccosum | 0,32 | - | 0,09 | 0,02 |
| T. tonsurans | 0,19 | - | 0,90 | 1,20 |

### EXAMPLE 5

### In vivo tests of protection against experimentally-induced dermatophytosis in the guinea pig, caused by Trichophyton mentagrophytes.

Two double-blind comparative tests were carried out, with the following results:

| In vivo protection against dermatophytosis | |
|---|---|
| Product | % of negative cultures after treatment |
| WAS 2160 | 50 % |
| Bifonazole | 39 % (test I) |
| Was 2169 | 81,8% |
| Bifonazole | 0 % (test II) |

### EXAMPLE 6

### In vivo test of antifungal activity against candidiosis in guinea pig

A double-blind comparative test was carried out with WAS 2160 and Clotrimazole, with the following results:

| In vivo protection against candidiosis | | |
|---|---|---|
| Product tested | % of negative cultures after treatment | % of animals with total clin. healing |
| WAS 2160 | 100 % | 60 % |
| Clotrimazole | 100 % | 30 % |

### EXAMPLE 7

### In vitro test against 30 strains of Malassezia furfur (Pytiosporum)

A double-blind comparative test was carried out with WAS 2160, Clotrimazole and Bifonazole, with the results of the table. (PIC = Partial Inhibitory Concentration: growth inhibition greater than 50% in relation to controls).

| In vitro protection against Malassezia fufur | | |
|---|---|---|
| Product tested | % of strains with MIC lower than 40 »g/mL | % of strains with PIC lower than 40 »g/mL |
| WAS 2160 | 40 % | 60 % |
| Clotrimazole | 33 % | 56 % |
| Bifonazole | 83 % | 50 % |

### EXAMPLE 8

### Tolerance test of WAS 2160 in rabbits

Product WAS 2160 proved to be innocuous and totally tolerated by the eye, the skin and the vagina.

## Claims (Claims for the following Contracting State(s): AT, BE, CH, DE, DK, FR, GB, IT, LI, LU, NL, SE)

1. Dichloro-substituted 1-(5H-dibenzo[a,d]cyclohepten-5-yl)-1H-imidazole compounds of formula (I), where -X- is either a -CH₂-CH₂- or a -CH=CH- group, and pharmaceutically acceptable salts thereof.

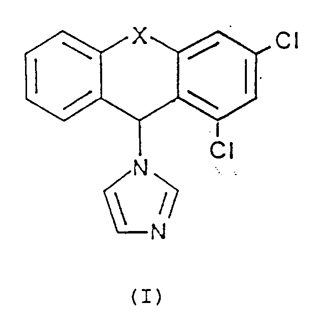

2. A pharmaceutical composition for treating fungal infections in humans and animals which comprises an antifungal effective amount of a compound of formula (I) in Claim 1 or a pharmaceutically acceptable salt thereof, in combination with a pharmaceutically acceptable excipient or carrier.

3. Use of a compound of formula (I) in Claim 1 or a pharmaceutically acceptable salt thereof in the preparation of a medicament for treating fungal infections in humans or animals.

4. The compound according to Claim 1 wherein -X- is -CH₂-CH₂-, such compound being 1-(2,4-dichloro-10,11-dihydro-5H-dibenzo[a,d]cyclohepten-5-yl)-1H-imidazole, or a pharmaceutical acceptable salt thereof.

5. A pharmaceutical composition for treating fungal infections in humans and animals according to Claim 2 which comprises an antifungal effective amount of 1-(2,4-dichloro-10,11-dihydro-5H-dibenzo[a,d]cyclohepten-5-yl)-1H-imidazole or a pharmaceutical acceptable salt thereof in combination with a pharmaceutically acceptable excipient or carrier.

6. The use according to Claim 3 wherein 1-(2,4-dichloro-10,11-dihydro-5H-dibenzo[a,d]cyclohepten-5-yl)-1H-imidazole or a pharmaceutical acceptable salt thereof is used in the preparation of a medicament for treating fungal infections in humans or animals.

7. The compound according to Claim 1 wherein -X- is -CH=CH-, such compound being 1-(2,4-dichloro-5H-dibenzo[a,d]cyclohepten-5-yl)-1H-imidazole, or a pharmaceutical acceptable salt thereof.

8. A pharmaceutical composition for treating fungal infections in humans and animals according to Claim 2 which comprises an antifungal effective amount of 1-(2,4-dichlcro-5H-dibenzo[a,d]cyclohepten-5-yl)-1H -imidazole or a pharmaceutical acceptable salt thereof in combination with a pharmaceutically acceptable excipient or carrier.

9. The use according to Claim 3 wherein 1-(2,4-dichloro-5H-dibenzo[a,d]cyclohepten-5-yl)-1H-imidazole or a pharmaceutical acceptable salt thereof is used in the preparation of a medicament for treating fungal infections in humans or animals.

10. A process for preparing a compound of formula (I) in Claim 1 or a pharmaceutically acceptable salt thereof, which comprises reacting a compound of formula (VI), where Z is a halogen group or a sulphonate group, with imidazole or an equivalent nucleophilic reactant selected from an imidazole salt or trimethylsilylimidazole, in a polar inert solvent, in the presence of a base to neutralise the acid formed; and, if a salt is desired, the subsequent addition of the corresponding acid.

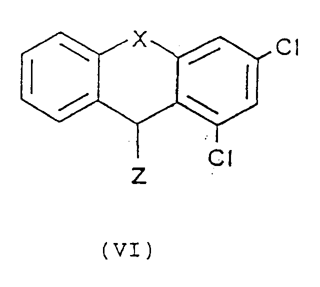

## Claims (Claims for the following Contracting State(s): ES, GR)

1. A process for preparing a compound of formula (I), where -X- is either a -CH₂-CH₂- or a -CH=CH-group, and pharmaceutically acceptable salts thereof, which comprises reacting a compound of formula (VI), where Z is a halogen group or a sulphonate group with imidazole or an equivalent nucleophilic reactant selected from an imidazole salt or trimethylsilylimidazole, in a polar inert solvent, in the presence of a base to neutralise the acid formed; and, if a salt is desired, the subsequent addition of the corresponding acid.

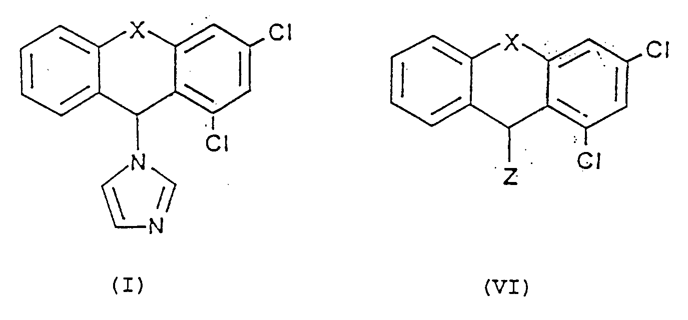

2. Process according to Claim 1 in which -X- is -CH₂ -CH₂- , wherein the obtained product is 1-(2,4-dichloro-10,11-dihydro-5H-dibenzo[a,d]-cyclohepten-5-yl)-1H-imidazole, and its pharmaceutically acceptable addition salts.

3. Process according to Claim 1 in which -X- is -CH=CH-, wherein the obtained product is 1-(2,4-dichloro-5H-dibenzo[a,d]cyclohepten-5-yl)-1H-imidazole or its pharmaceutically acceptable addition salts.

4. Process according to Claim 1 in which an excess of imidazole is used, without addition of another base.

5. Process according to Claim 1 in which the reaction is carried out in dimethylformamide.

6. Process according to Claim 1 in which the nitrate of (I) is prepared by addition of nitric acid.

7. Process according to Claim 1 in which the product of formula (VI) is prepared from the alcohol of formula (VII) by standard reagents.

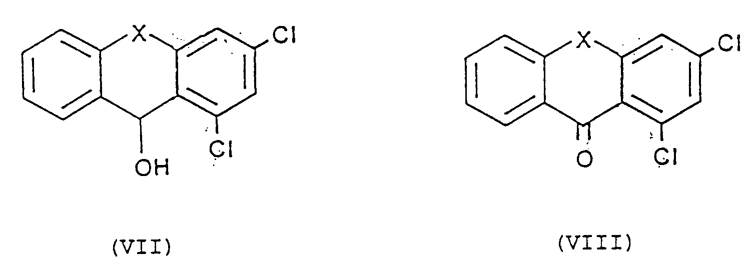

8. Process according to Claim 7 in which the alcohol of formula (VII) is prepared by reduction of the ketone of formula (VIII) by standard methods

## Patentansprüche (Patentansprüche für folgende(n) Vertragsstaat(en): AT, BE, CH, DE, DK, FR, GB, IT, LI, LU, NL, SE)

1. Dichlor-substituierte 1-(5H-Dibenzo[a,d] cyclohepten-5-yl)1H-imidazol-Verbindungen der Formel (I), worin -X- entweder eine -CH₂-CH₂- oder eine -CH=CH- Gruppe ist, und deren pharmazeutisch akzeptablen Salze.

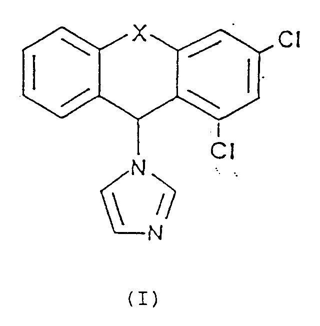

2. Pharmazeutische Zusammensetzung zur Behandlung von Pilzinfektionen bei Menschen und Tieren, welche eine gegen Pilze wirksame Menge einer Verbindung der Formel (I) nach Anspruch 1 oder ein pharmazeutisch akzeptables Salz derselben umfaßt, in Kombination mit einem pharmazeutisch akzeptablen Vehikel oder Träger.

3. Verwendung einer Verbindung der Formel (I) nach Anspruch 1 oder eines pharmazeutisch akzeptablen Salzes derselben bei der Herstellung eines Medikamentes für die Behandlung von Pilzinfektionen bei Menschen oder Tieren.

4. Verbindung nach Anspruch 1, worin -X- -CH₂-CH₂-bedeutet, wobei diese Verbindung 1-(2,4-Dichlor-10,11-dihydro-5H-dibenzo[a,d]cyclohepten-5-yl)-1H- imidazol oder ein pharmazeutisch akzeptables Salz desselben ist.

5. Pharmazeutische Zusammensetzung für die Behandlung von Pilzinfektionen bei Menschen und Tieren nach Anspruch 2, welche eine gegen Pilze wirksame Menge von 1-(2,4-Dichlor-10,11-dihydro-5H-dibenzo[a,d]cyclohepten-5-yl)-1H-imidazol oder ein pharmazeutisch akzeptables Salz desselben in Kombination mit einem pharmazeutisch akzeptablen Vehikel oder Träger umfaßt.

6. Verwendung nach Anspruch 3, wobei 1-(2,4-Dichlor-10,11-dihydro-5H-dibenzo[a,d]cyclohepten-5-yl)-1H-imidazol oder ein pharmazeutisch akzeptables Salz desselben bei der Herstellung eines Medikamentes für die Behandlung von Pilzinfektionen bei Menschen oder Tieren verwendet wird.

7. Verbindung nach Anspruch 1, worin -X- -CH=CH- bedeutet, wobei diese Verbindung 1-(2,4-Dichlor-5H-dibenzo[a,d]cyclohepten-5-yl)-1H-imidazol oder ein pharmazeutisch akzeptables Salz desselben ist.

8. Pharmazeutische Zusammensetzung für die Behandlung von Pilzinfektionen bei Menschen und Tieren nach Anspruch 2, welche eine gegen Pilze wirksame Menge von 1-(2,4-Dichlor-5H-dibenzo[a,d]cyclohepten-5-yl)-1H-imidazol oder ein pharmazeutisch akzeptables Salz desselben in Kombination mit einem pharmazeutisch akzeptablen Vehikel oder Träger umfaßt.

9. Verwendung nach Anspruch 3, wobei 1-(2,4-Dichlor-5H-dibenzo[a,d]cyclohepten-5-yl)-1H-imidazol oder ein pharmazeutisch akzeptables Salz desselben bei der Herstellung eines Medikamentes für die Behandlung von Pilzinfektionen bei Menschen oder Tieren verwendet wird.

10. Verfahren zur Herstellung einer Verbindung der Formel (I) nach Anspruch 1 oder eines pharmazeutisch akzeptablen Salzes derselben, welches umfaßt: Herstellen einer Reaktion zwischen einer Verbindung der Formel (VI), worin Z eine Halogengruppe oder eine Sulfonatgruppe ist, und Imidazol oder einem äquivalenten nucleophilen Reaktanten, ausgewählt aus einem Imidazolsalz oder Trimethylsilylimidazol, in einem polaren inerten Lösungsmittel in Gegenwart einer Base, zum Neutralisieren der gebildeten Säure; und, wenn ein Salz gewünscht ist, die darauffolgende Zugabe der entsprechenden Säure.

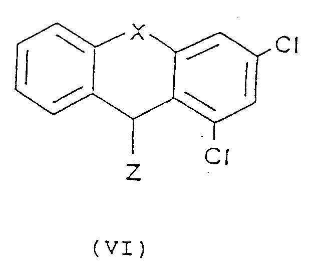

## Patentansprüche (Patentansprüche für folgende(n) Vertragsstaat(en): ES, GR)

1. Verfahren zur Herstellung einer Verbindung der Formel (I), worin -X- entweder eine -CH₂-CH₂- oder eine -CH=CH-Gruppe ist, und eines pharmazeutisch akzeptablen Salzes derselben, welches umfaßt: Herstellen einer Reaktion zwischen einer Verbindung der Formel (VI), worin Z eine Halogengruppe oder eine Sulfonatgruppe ist, und Imidazol oder einem äquivalenten nucleophilen Reaktanten, ausgewählt aus einem Imidazolsalz oder Trimethylsilylimidazol, in einem polaren inerten Lösungsmittel in Gegenwart einer Base, zum Neutralisieren der gebildeten Säure und, wenn ein Salz gewünscht ist, die darauffolgende Zugabe der entsprechenden Säure.

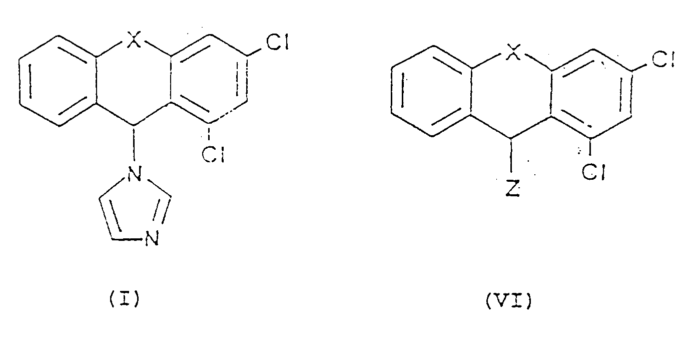

2. Verfahren nach Anspruch 1, worin -X- -CH₂-CH₂-bedeutet, wobei das erhaltene Produkt 1-(2,4-Dichlor-10,11-dihydro-5H-dibenzo[a,d]-cyclohepten-5-yl)-1H-imidazol und pharmazeutisch akzeptable Additionssalze desselben ist.

3. Verfahren nach Anspruch 1, worin -X- -CH=CH-bedeutet, wobei das erhaltene Produkt 1-(2,4-Dichlor-5H-dibenzo[a,d]cyclohepten-5-yl)-1H- imidazol oder pharmazeutisch akzeptable Additionssalze desselben ist.

4. Verfahren nach Anspruch 1, wobei ein Überschuß an Imidazol ohne die Zugabe einer anderen Base verwendet wird.

5. Verfahren nach Anspruch 1, wobei die Reaktion in Dimethylformamid durchgeführt wird.

6. Verfahren nach Anspruch 1, wobei das Nitrat von (I) durch Zugabe von Salpetersäure hergestellt wird.

7. Verfahren nach Anspruch 1, wobei das Produkt der Formel (VI) aus dem Alkohol der Formel (VII) mit Hilfe von Standardreagenzien hergestellt wird.

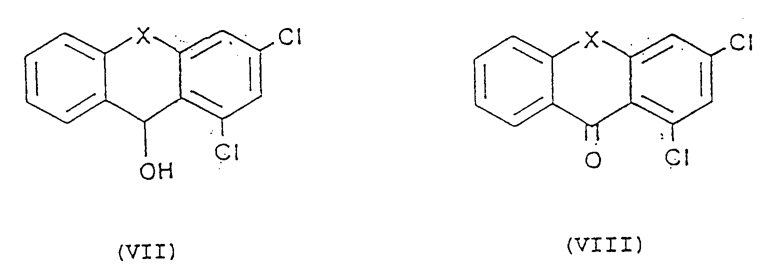

8. Verfahren nach Anspruch 7, wobei der Alkohol der Formel (VII) durch Reduktion des Ketons der Formel (VIII) mit Hilfe von Standardmethoden hergestellt wird.

## Revendications (Revendications pour l'(les) Etat(s) contractant(s) suivant(s): AT, BE, CH, DE, DK, FR, GB, IT, LI, LU, NL, SE)

1. Composés 1-(5H-dibenzo[a,d]cycloheptène-5-yl)-1H-imidazole dichloro substitués de formule (I), où -X-est un groupe -CH₂-CH₂- ou -CH=CH-, et les sels pharmaceutiquement acceptable de ceux-ci.

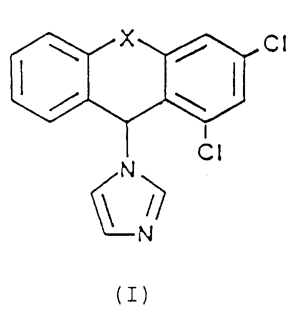

2. Composition pharmaceutique pour traiter des infections fongiques chez les humains et les animaux, qui comprend une quantité antifongique efficace d'un composé de formule (I) selon la revendication 1, ou un sel pharmaceutiquement acceptable de celui-ci, en combinaison avec un excipient ou un support pharmaceutiquement acceptable.

3. Utilisation d'un composé de formule (I) selon la revendication 1 ou d'un sel pharmaceutiquement acceptable de celui-ci, dans la préparation d'un médicament pour traiter des infections fongiques chez les humains ou les animaux.

4. Composé selon la revendication 1, dans lequel -X-est -CH₂-CH₂-, ce composé étant le 1-(2,4-dichloro-10,11-dihydro-5H-dibenzo[a,d]cycloheptène-5-yl)-1H-imidazole, ou un sel pharmaceutique acceptable de celui-ci.

5. Composition pharmaceutique pour traiter des infections fongiques chez les humains et les animaux selon la revendication 2, qui comprend une quantité antifongique efficace de 1-(2,4-dichloro-10,11-dihydro-5H-dibenzo[a,d]cycloheptène-5-yl)-1H-imidazole, ou un sel pharmaceutique acceptable de celui-ci, en combinaison avec un excipient ou un support pharmaceutiquement acceptable.

6. Utilisation selon la revendication 3, dans laquelle le 1-(2,4-dichloro-10,11-dihydro-5H-dibenzo-[a,d]cycloheptène-5-yl)-1H-imidazole, ou un sel pharmaceutique acceptable de celui-ci, est utilisé dans la préparation d'un médicament pour traiter des infections fongiques chez les humains ou les animaux.

7. Composé selon la revendication 1, dans lequel -X-est -CH=CH-, ce composé étant le 1-(2,4-dichloro-5H-dibenzo[a,d]cycloheptène-5-yl)-1H-imidazole, ou un sel pharmaceutique acceptable de celui-ci.

8. Composition pharmaceutique pour traiter des infections fongiques chez les humains et les animaux selon la revendication 2, qui comprend une quantité antifongique efficace de 1-(2,4-dichloro-5H-dibenzo-[a,d]cycloheptène-5-yl)-1H-imidazole, ou un sel pharmaceutique acceptable de celui-ci, en combinaison avec un excipient ou un support pharmaceutiquement acceptable.

9. Utilisation selon la revendication 3, dans laquelle le 1-(2,4-dichloro-5H-dibenzo[a,d]cycloheptène-5-yl)-1H-imidazole, ou un sel pharmaceutique acceptable de celui-ci, est utilisé dans la préparation d'un médicament pour traiter des infections fongiques chez les humains ou les animaux.

10. Procédé pour préparer un composé de formule (I) selon la revendication 1, ou un sel pharmaceutiquement acceptable de celui-ci, qui comprend la réaction d'un composé de formule (VI), où Z est un groupe halogène ou un groupe sulfonate, avec un imidazole ou un réactif nucléophile équivalent, choisi parmi un sel d'imidazole ou le triméthylsilylimidazole, dans un solvant polaire inerte, en présence d'une base, pour neutraliser l'acide formé ; et, si un sel est désiré, l'addition subséquente de l'acide correspondant.

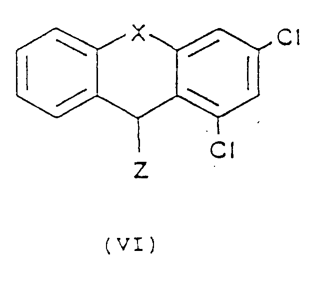

## Revendications (Revendications pour l'(les) Etat(s) contractant(s) suivant(s): ES, GR)

1. Procédé pour préparer un composé de formule (I), où -X- est un groupe -CH₂-CH₂- ou -CH=CH-, et les sels pharmaceutiquement acceptables de celui-ci, qui comprend la réaction d'un composé de formule (VI), où Z est un groupe halogène ou un groupe sulfonate, avec de l'imidazole ou un réactif nucléophile équivalent choisi parmi un sel d'imidazole ou le triméthylsilylimidazole, dans un solvant polaire inerte, en présence d'une base, pour neutraliser l'acide formé ; et, si un sel est désiré, l'addition subséquente de l'acide correspondant.

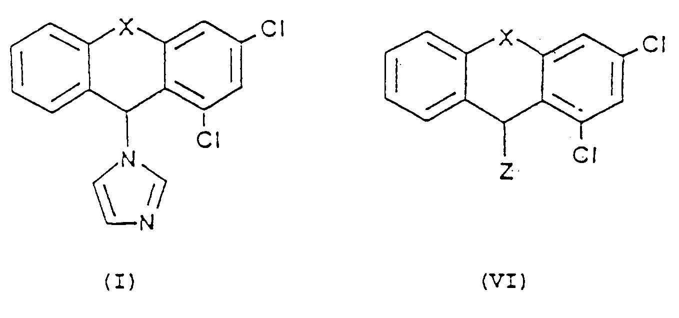

2. Procédé selon la revendication 1, dans lequel -X-est -CH₂-CH₂-, où le produit obtenu est le 1-(2,4-dichloro-10,11-dihydro-5H-dibenzo[a,d]cycloheptène-5-yl)-1H-imidazole, et ses sels d'addition pharmaceutiquement acceptables.

3. Procédé selon la revendication 1, dans lequel -X-est -CH=CH-, où le produit obtenu est le 1-(2,4-dichloro-5H-dibenzo[a,d]cycloheptène-5-yl)-1H-imidazole, ou ses sels d'addition pharmaceutiquement acceptables.

4. Procédé selon la revendication 1, dans lequel on utilise un excès d'imidazole, sans addition d'une autre base.

5. Procédé selon la revendication 1, dans lequel la réaction est effectuée dans du diméthylformamide.

6. Procédé selon la revendication 1, dans lequel le nitrate de (I) est préparé par addition d'acide nitrique.

7. Procédé selon la revendication 1, dans lequel le produit de formule (VI) est préparé à partir de l'alcool de formule (VII) par des réactifs standards.

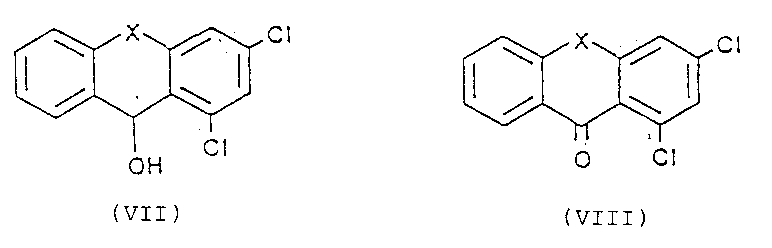

8. Procédé selon la revendication 7, dans lequel l'alcool de formule (VII) est préparé par réduction de la cétone de formule (VIII) par des procédés standards.
